(19) **Europäisches Patentamt / European Patent Office / Office européen des brevets**

(11) **EP 3 943 109 A1**

(12) **EUROPEAN PATENT APPLICATION**

(43) Date of publication:
**26.01.2022 Bulletin 2022/04**

(21) Application number: **21179229.6**

(22) Date of filing: **15.09.2015**

(51) International Patent Classification (IPC):
$A61K\ 39/395^{(2006.01)}$    $A61K\ 31/519^{(2006.01)}$
$C07K\ 16/28^{(2006.01)}$    $A61K\ 31/42^{(2006.01)}$
$A61K\ 31/655^{(2006.01)}$    $A61K\ 45/06^{(2006.01)}$
$A61K\ 39/00^{(2006.01)}$    $A61P\ 29/00^{(2006.01)}$
$A61P\ 19/02^{(2006.01)}$    $A61P\ 43/00^{(2006.01)}$
$A61P\ 37/06^{(2006.01)}$    $A61K\ 31/4706^{(2006.01)}$

(52) Cooperative Patent Classification (CPC):
(C-Sets available)
A61K 31/42; A61K 31/4706; A61K 31/519;
A61K 31/655; A61K 39/39541; A61K 45/06;
A61P 19/02; A61P 29/00; A61P 37/06;
A61P 43/00; C07K 16/2866; A61K 2039/505

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **16.09.2014 EP 14306427**

(62) Document number(s) of the earlier application(s) in accordance with Art. 76 EPC:
**15779060.1 / 3 193 934**

(71) Applicants:
• **Sanofi Biotechnology**
  **75008 Paris (FR)**
• **Regeneron Pharmaceuticals, Inc.**
  **Tarrytown, NY 10591 (US)**

(72) Inventors:
• **JOSEPH, George Johny**
  **BRIDGEWATER, 08807-155 (US)**
• **VAN HOOGSTRATEN, Hubert**
  **BRIDGEWATER, 08807 (US)**

• **CHEN,, Chieh-I**
  **TARRYTOWN, 10591 (US)**
• **FAN, Chungpeng**
  **BRIDGEWATER, 08807 (US)**
• **MOMTAHEN, Tanya Marie**
  **BRIDGEWATER, 08807 (US)**
• **GENOVESE, Mark**
  **SUNNYVALE, 94087 (US)**
• **GRAHAM, Neil**
  **TARRYTOWN, 10591 (US)**

(74) Representative: **Lavoix**
**Bayerstraße 83**
**80335 München (DE)**

Remarks:
•This application was filed on 14-06-2021 as a divisional application to the application mentioned under INID code 62.
•Claims filed after the date of filing of the application / after the date of receipt of the divisional application (Rule 68(4) EPC).

(54) **COMPOSITION FOR IMPROVING THE HEALTH RELATED QUALITY OF LIFE OF RHEUMATOID ARTHRITIS PATIENTS**

(57) The present invention provides compositions and methods of for improving the Health-Related Quality of Life of a subject suffering from rheumatoid arthritis, comprising administering to the subject an effective amount of sarilumab.

**(Cont. next page)**

(52) Cooperative Patent Classification (CPC): (Cont.)

C-Sets
**A61K 31/42, A61K 2300/00;**
**A61K 31/4706, A61K 2300/00;**
**A61K 31/519, A61K 2300/00;**
**A61K 31/655, A61K 2300/00;**
**A61K 39/39541, A61K 2300/00**

**Description**

**FIELD OF THE INVENTION**

The present invention relates to the field of rheumatoid arthritis. More specifically, the invention relates to methods of improving the Health-Related Quality of Life of subjects suffering from rheumatoid arthritis, comprising administering to the subjects an effective amount of sarilumab.

**BACKGROUND**

It is estimated that approximately 0.5% to 1% of the adult population in North America and Europe is affected by rheumatoid arthritis (RA). RA affects women twice as often as men and the incidence is highest among women over 40 years of age.

RA is characterized by persistent synovitis and progressive destruction of cartilage and bone in multiple joints. The hallmark of the disease is a symmetric polyarthritis characteristically involving the small joints of the hands and feet. The inflammatory process can also target other organs, characteristically bone marrow (anemia), eye (scleritis, episcleritis), lung (interstitial pneumonitis, pleuritis), cardiac (pericarditis) and skin (nodules, leukocytoclastic vasculitis). Systemic inflammation is characterized by laboratory abnormalities, such as anemia, elevated erythrocyte sedimentation rate, fibrinogen and C-reactive protein (CRP) and by clinical symptoms of fatigue, weight loss, muscle atrophy in affected joint areas. The presence of polyclonal high-titer rheumatoid factors and anticyclic citrullinated peptide (anti-CCP) antibodies provides evidence of immune dysregulation. It has been estimated that 65% to 70% of RA patients have progressive disease that leads to joint destruction, disability and premature death.

In addition to improving the clinical symptoms of RA patients, there has been a growing interest in the improvement of the health related quality of life of RA patients. Indeed, in addition to the usual instruments intended to assess health status of the patients (presence or absence of disease and its consequences), physicians and clinicians developed new instruments to measure the quality of life of the patients. Quality of life goes beyond the impairment/disability and handicap continuum by asking what patients' health status prevents them from doing and also about their emotional response to these restrictions. Quality of life also reflects the influences of the personal, social and economic resources that an individual has and the way in which these interact with health status (British Journal of Rheumatology 1997;36:884-888).

**SUMMARY**

The present disclosure provides methods of improving the Health-Related Quality of Life of a subject suffering from rheumatoid arthritis, comprising administering to the subject an effective amount of sarilumab (SAR153191).

Examples of embodiments of the invention are listed below:

Embodiment 1:

A method of improving the Health-Related Quality of Life of a subject suffering from rheumatoid arthritis, comprising administering to the subject an effective amount of sarilumab.

Embodiment 2:

The method according to Embodiment 1, wherein the subject achieves after at least 24 weeks of treatment a change from baseline (BL) in any one or more Health-Related Quality of Life outcomes selected from the group consisting of:

- a Short Form-36 Physical Component Summary (SF-36 PCS) change from BL of at least 2.5,
- a Short Form-36 Mental Component Summary (SF-36 MCS) change from BL of at least 2.5,
- eight domains of the SF-36:

  ◦ a Short Form-36 Physical Functioning (SF-36 PF) change from BL of at least 5,
  ◦ a Short Form-36 Role Physical (SF-36 RP) change from BL of at least 5,
  ◦ a Short Form-36 Body Pain (SF-36 BP) change from BL of at least 5,
  ◦ a Short Form-36 General Health (SF-36 GH) change from BL of at least 5,
  ◦ a Short Form-36 Vitality (SF-36 VT) change from BL of at least 5,

◦ a Short Form-36 Social Functioning (SF-36 SF) change from BL of at least 5,
◦ a Short Form-36 Role Emotional (SF-36 RE) change from BL of at least 5,
◦ a Short Form-36 Mental Health (SF-36 MH) change from BL of at least 5,

- a Functional Assessment of Chronic Illness Therapy-Fatigue (FACIT-F) change from BL of at least 3.

Embodiment 3:

The method according to embodiment 1 or 2, wherein the subject achieves after at least 24 weeks of treatment with sarilumab a change from baseline (BL) greater than the change from BL obtained after at least 24 weeks of treatment with placebo in all eight domains of the SF-36, as well as in SF-36 PCS, SF-36 MCS.

Embodiment 4:

The method according to embodiment 3, wherein the change from baseline (BL) greater than the change from BL obtained after at least 24 weeks of treatment with placebo in all eight domains of the SF-36, as well as in SF-36 PCS, SF-36 MCS, consists of:

- a SF-36 PCS change from BL of at least 5.3,
- a SF-36 MCS change from BL of at least 4,
- a SF-36 PF change from BL of at least 5,
- a SF-36 RP change from BL of at least 5,
- a SF-36 BP change from BL of at least 6.6,
- a SF-36 GH change from BL of at least 5,
- a SF-36 VT change from BL of at least 5.5,
- a SF-36 SF change from BL of at least 5,
- a SF-36 RE change from BL of at least 5,
- a SF-36 MH change from BL of at least 5,
- a FACIT-F change from BL of at least 6.5.

Embodiment 5:

The method according to embodiment 1 or 2, wherein the subject achieves after at least 24 weeks of treatment a change from baseline (BL) in any one or more Health-Related Quality of Life outcomes selected from the group consisting of:

- a SF-36 PCS change from BL of at least 5.3,
- a SF-36 MCS change from BL of at least 4,
- a SF-36 PF change from BL of at least 5,
- a SF-36 RP change from BL of at least 5,
- a SF-36 BP change from BL of at least 6.6,
- a SF-36 GH change from BL of at least 5,
- a SF-36 VT change from BL of at least 5.5,
- a SF-36 SF change from BL of at least 5,
- a SF-36 RE change from BL of at least 5,
- a SF-36 MH change from BL of at least 5,
- a FACIT-F change from BL of at least 6.5.

Embodiment 6:

The method according to any one of embodiments 1 to 5, wherein the subject achieves after at least 24 weeks of treatment a score in any one or more Health-Related Quality of Life outcomes selected from the group consisting of:

- a SF-36 PCS change from baseline (BL) of at least 8,
- a SF-36 MCS change from BL of at least 5,
- a SF-36 PF change from BL of at least 7,
- a SF-36 RP change from BL of at least 7,
- a SF-36 BP change from BL of at least 10,

- a SF-36 GH change from BL of at least 6,
- a SF-36 VT change from BL of at least 7,
- a SF-36 SF change from BL of at least 7,
- a SF-36 RE change from BL of at least 6,
- a SF-36 MH change from BL of at least 5,
- a FACIT-F change from BL of at least 9.

Embodiment 7:

The method of any one of embodiments 1 to 6, wherein the subject was previously ineffectively treated for rheumatoid arthritis by administering at least one disease-modifying anti-rheumatic drug (DMARD).

Embodiment 8:

The method of embodiment 7, wherein the at least one DMARD is selected from the group consisting of methotrexate, leflunomide, sulfasalazi ne, hydroxychloroquine, abatacept, rituximab, tocilizumab, adalimumab, certolizumab pegol, etanercept, golimumab and infliximab.

Embodiment 9:

The method of embodiment 7, wherein the at least one DMARD is selected from the group consisting of methotrexate, leflunomide, sulfasalazine and hydroxychloroquine.

Embodiment 10:

The method of embodiment 7, wherein the at least one DMARD is selected from the group consisting of adalimumab, certolizumab pegol, etanercept, golimumab and infliximab.

Embodiment 11:

The method of any of embodiments 1 to 10, wherein the method comprises administering to the subject an effective amount of sarilumab and an effective amount of methotrexate, leflunomide, sulfasalazine and/or hydroxychloroquine.

Embodiment 12:

The method of any of embodiments 1 to 11, wherein the method comprises administering to the subject an effective amount of sarilumab and an effective amount of methotrexate.

Embodiment 13:

The method of embodiment 12, wherein methotrexate is administered between 6 to 25 mg per week.

Embodiment 14:

The method of any of embodiments 1 to 13, wherein sarilumab is administered subcutaneously.

Embodiment 15:

The method of any of embodiments 1 to 14, wherein sarilumab is administered at 150 mg per two weeks or at 200 mg per two weeks.

Embodiment 16:

Sarilumab for use in a method of improving the Health-Related Quality of Life of a subject suffering from rheumatoid arthritis.

Embodiment 17:

Sarilumab for use according to embodiment 16, wherein the subject achieves after at least 24 weeks of treatment a change from baseline (BL) in any one or more Health-Related Quality of Life outcomes selected from the group consisting of:

- a Short Form-36 Physical Component Summary (SF-36 PCS) change from BL of at least 2.5,
- a Short Form-36 Mental Component Summary (SF-36 MCS) change from BL of at least 2.5,
- eight domains of the SF-36:

  ∘ a Short Form-36 Physical Functioning (SF-36 PF) change from BL of at least 5,
  ∘ a Short Form-36 Role Physical (SF-36 RP) change from BL of at least 5,
  o a Short Form-36 Body Pain (SF-36 BP) change from BL of at least 5,
  ∘ a Short Form-36 General Health (SF-36 GH) change from BL of at least 5,
  ∘ a Short Form-36 Vitality (SF-36 VT) change from BL of at least 5, o a Short Form-36 Social Functioning (SF-36 SF) change from BL of at least 5,
  ∘ a Short Form-36 Role Emotional (SF-36 RE) change from BL of at least 5,
  ∘ a Short Form-36 Mental Health (SF-36 MH) change from BL of at least 5,

- a Functional Assessment of Chronic Illness Therapy-Fatigue (FACIT-F) change from BL of at least 3.

Embodiment 18:

Sarilumab for use according to embodiment 16 or 17, wherein the subject achieves after at least 24 weeks of treatment with sarilumab a change from baseline (BL) greater than the change from BL obtained after at least 24 weeks of treatment with placebo in all eight domains of the SF-36, as well as in SF-36 PCS, SF-36 MCS.

Embodiment 19:

Sarilumab for use according to embodiment 18, wherein the change from baseline (BL) greater than the change from BL obtained after at least 24 weeks of treatment with placebo in all eight domains of the SF-36, as well as in SF-36 PCS, SF-36 MCS, consists of:

- a SF-36 PCS change from BL of at least 5.3,
- a SF-36 MCS change from BL of at least 4,
- a SF-36 PF change from BL of at least 5,
- a SF-36 RP change from BL of at least 5,
- a SF-36 BP change from BL of at least 6.6,
- a SF-36 GH change from BL of at least 5,
- a SF-36 VT change from BL of at least 5.5,
- a SF-36 SF change from BL of at least 5,
- a SF-36 RE change from BL of at least 5,
- a SF-36 MH change from BL of at least 5,
- a FACIT-F change from BL of at least 6.5.

Embodiment 20:

Sarilumab for use according to embodiment 16 or embodiment 17, wherein the subject achieves after at least 24 weeks of treatment a change from baseline (BL) in any one or more Health-Related Quality of Life outcomes selected from the group consisting of:

- a SF-36 PCS change from BL of at least 5.3,
- a SF-36 MCS change from BL of at least 4,
- a SF-36 PF change from BL of at least 5,
- a SF-36 RP change from BL of at least 5,
- a SF-36 BP change from BL of at least 6.6,

- a SF-36 GH change from BL of at least 5,
- a SF-36 VT change from BL of at least 5.5,
- a SF-36 SF change from BL of at least 5,
- a SF-36 RE change from BL of at least 5,
- a SF-36 MH change from BL of at least 5,
- a FACIT-F change from BL of at least 6.5.

Embodiment 21:

Sarilumab for use according to any of embodiments 16 to 20, wherein the subject achieves after at least 24 weeks of treatment a score in any one or more Health-Related Quality of Life outcomes selected from the group consisting of:

- a SF-36 PCS change from baseline (BL) of at least 8,
- a SF-36 MCS change from BL of at least 5,
- a SF-36 PF change from BL of at least 7,
- a SF-36 RP change from BL of at least 7,
- a SF-36 BP change from BL of at least 10,
- a SF-36 GH change from BL of at least 6,
- a SF-36 VT change from BL of at least 7,
- a SF-36 SF change from BL of at least 7,
- a SF-36 RE change from BL of at least 6,
- a SF-36 MH change from BL of at least 5,
- a FACIT-F change from BL of at least 9.

Embodiment 22:

Sarilumab for use according to any one of Embodiments 16 to 21, wherein the subject was previously ineffectively treated for rheumatoid arthritis by administering at least one disease-modifying anti-rheumatic drug (DMARD).

Embodiment 23

Sarilumab for use according to Embodiment 22, wherein the at least one DMARD is selected from the group consisting of methotrexate, leflunomide, sulfasalazine, 15 hydroxychloroquine, abatacept, rituximab, tocilizumab, adalimumab, certolizumab pegol, etanercept, golimumab and infliximab.

Embodiment 24:

Sarilumab for use according to Embodiment 22, wherein the at least one DMARD is selected from the group consisting of methotrexate, leflunomide, sulfasalazine and hydroxychloroquine.

Embodiment 25:

Sarilumab for use according to Embodiment 22, wherein the at least one DMARD is selected from the group consisting of adalimumab, certolizumab pegol, etanercept, golimumab and infliximab.

Embodiment 26:

Sarilumab for use according to any of Embodiments 16 to 25, wherein the method comprises administering to the subject an effective amount of sarilumab and an effective amount of methotrexate, leflunomide, sulfasalazine and/or hydroxychloroquine.

Embodiment 27:

Sarilumab for use according to any of Embodiments 16 to 26, wherein the method comprises administering to the subject an effective amount of sarilumab and an effective amount of methotrexate.

Embodiment 28:

Sarilumab for use according to Embodiment 27, wherein methotrexate is administered between 6 to 25 mg per week.

Embodiment 29:

Sarilumab for use according to any of Embodiments 16 to 28, wherein sarilumab is administered subcutaneously.

Embodiment 30:

Sarilumab for use according to any of Embodiments 16 to 29, wherein sarilumab is administered at 150 mg per two weeks or at 200 mg per two weeks.

Embodiment 31:

The method according to any of embodiments 1 to 15, or sarilumab for use according to any of Embodiments 16 to 30, wherein sarilumab is administered subcutaneously, in an aqueous buffered solution at pH 6.0 containing:

- 25 mM histidine
- 50 mM arginine
- 0.2% (w/v) polysorbate 20, and
- 5% (w/v) sucrose
- between 100 mg/mL and 200 mg/mL sarilumab.

## DETAILED DESCRIPTION

It has been shown by the inventors that in addition to treating RA, sarilumab was also effective for improving the Quality of Life of subjects suffering from rheumatoid arthritis.

The present disclosure thus provides methods of improving the Health-Related Quality of Life of a subject suffering from rheumatoid arthritis, comprising administering to the subject an effective amount of sarilumab (SAR153191).

The improvement of Health-Related Quality of Life of a subject suffering from rheumatoid arthritis is assessed via Health-Related Quality of Life scores, which are typically selected from the group consisting of:

- the Short Form-36 Physical Component Summary (SF-36 PCS),
- the Short Form-36 Mental Component Summary (SF-36 MCS),
- the eight domains of the SF-36:

  ○ the Short Form-36 Physical Functioning (SF-36 PF),
  ○ a Short Form-36 Role Physical (SF-36 RP),
  ○ a Short Form-36 Body Pain (SF-36 BP),
  ○ a Short Form-36 General Health (SF-36 GH),
  ○ a Short Form-36 Vitality (SF-36 VT),
  ○ a Short Form-36 Social Functioning (SF-36 SF),
  ○ a Short Form-36 Role Emotional (SF-36 RE),
  ○ a Short Form-36 Mental Health (SF-36 MH),

- a Functional Assessment of Chronic Illness Therapy-Fatigue (FACIT-F)
- WPAI,
- Sleep VAS.

Improvement of Health-Related Quality of Life of a subject suffering from rheumatoid arthritis, following administration of sarilumab according to the invention, is typically assessed by looking at a change from baseline (BL) in each of said Health-Related Quality of Life scores.

Baseline is defined as the score obtained by the subject before being administered with sarilumab according to the invention.

Change from baseline is defined as the difference existing between the score obtained by the subject at baseline and the score obtained by the subject after being administered with sarilumab according to the invention, typically measured at least 24 weeks after the first administration of sarilumab, particularly at 24 weeks or at 52 weeks after the first administration of sarilumab.

### *Health related quality of life scores*

SF-36 V2

QualityMetric's SF-36v2[®] Health Survey asks 36 questions to measure functional health and well-being from the patient's point of view. It is a practical, reliable, and valid measure of physical and mental health that can be completed in five to ten minutes by the patient, and which is recognized by both the scientific community and the health regulatory agencies. All the necessary material & guidance to measure the SF-36 scores are provided by QualityMetric (Lincoln, RI, USA). The SF-36 V2 yields scores for eight domains (Physical Functioning, Role-Physical, Bodily pain, General health, Vitality, Social Functioning, Role-Emotional, and Mental Health, each scale is scored from 0 to 100 where higher scores indicate better health and well-being), as well as two summary measures of physical and mental health: the Physical Component Summary and Mental Component Summary (Ware, JE.; Kosinski, M.; Keller, SD. SF-36 Physical and Mental Health Summary Scales: A User's Manual. Boston, MA: The Health Institute; 1994). The scoring process is summarized below:

1. Enter item response data.
2. Recode item response values.
3. Determine health domain scale raw scores.
4. Transform health domain scale raw scores to 0-100 scores.
5. 0-100 raw scores are converted into Z-Transformed scores for each of the 8 domains, using the following conversion formulae:

$$pf\_z=(pf-83.29094)/23.75883;$$

$$rp\_z=(rp-82.50964)/25.52028;$$

$$bp\_z=(bp-71.32527)/23.66224;$$

$$gh\_z=(gh-70.84570)/20.97821;$$

$$vt\_z=(vt-58.31411)/20.01923;$$

$$sf\_z=(sf-84.30250)/22.91921;$$

$$re\_z=(re-87.39733)/21.43778;$$

$$mh\_z=(mh-74.98685)/17.75604;$$

5. Transform Z-transformed health domain scores to norm-based scores, as
6. Score physical and mental component summary measures:

I) Using a specific weighted formula (see below), the 8 z-transformed health domain scores are used to compute the PCS score. This aggregate PCS score is converted to norm-based PCS score:

$$AGG\_PHYS=(PF\_Z * .42402) + (RP\_Z * .35119) + (BP\_Z * .31754) + (GH\_Z * .24954) + (VT\_Z * .02877) + (SF\_Z * -.00753) + (RE\_Z * -.19206) + (MH\_Z * -.22069);$$

II) Using a different specific weighted formula (see below), the 8 z-transformed health domain scores are used to compute the MCS score. This aggregate MCS score is converted to norm-based MCS score:$AGG\_MENT=(PF\_Z * -.22999) + (RP\_Z * -.12329) + (BP\_Z * -.09731) + (GH\_Z * -.01571) + (VT\_Z * .23534) + (SF\_Z * .26876) + (RE\_Z * .43407) + (MH\_Z * .48581);$

The following table shows the construction and summary measures of the SF-36 scales:

**Table: SF-36 V2 measurement model**

| Summary Measures | Scales | Items |
|---|---|---|
| Physical ComponentSummary (PCS)* | Physical Functioning (PF) | 3a, 3b, 3c, 3d, 3e, 3f, 3g, 3h, 3i, 3j |
| | Role-Physical (RP) | 4a, 4b, 4c, 4d |
| | Bodily Pain (BP) | 7, 8 |
| | General Health (GH) | 1, 11a, 11b, 11c, 11d |

(continued)

| Summary Measures | Scales | Items |
|---|---|---|
| Mental Component Summary (MCS)* | Vitality (VT) | 9a, 9e, 9g, 9i |
| | Social Functioning (SF) | 6, 10 |
| | Role-Emotional (RE) | 5a, 5b, 5c |
| | Mental Health (MH) | 9b, 9c, 9d, 9f, 9h |

*MCS and PCS derived from the eight scales (Ware, JE. et al. 1994) The Abbreviated Item Content is as follows:
3a Vigorous activities, such as running, lifting heavy objects, or participating in strenuous sports
3b Moderate activities, such as moving a table, pushing a vacuum cleaner, bowling, or playing golf
3c Lifting or carrying groceries
3d Climbing several flights of stairs
3e Climbing one flight of stairs
3f Bending, kneeling, or stooping
3g Walking more than a mile
3h Walking several hundred yards
3i Walking one hundred yards
3j Bathing or dressing oneself
4a Cut down the amount of time one spent on work or other activities
4b Accomplished less than you would like
4c Limited in kind of work or other activities
4d Had difficulty performing work or other activities (e.g., it took extra effort)
7 Intensity of bodily pain
8 Extent pain interfered with normal work
1 Is your health: excellent, very good, good, fair, poor
11a Seem to get sick a little easier than other people
11b As healthy as anybody I know
11c Expect my health to get worse
11d Health is excellent
9a Feel full of life
9e Have a lot of energy
9g Feel worn out
9i Feel tired
6 Extent health problems interfered with normal social activities
10 Frequency health problems interfered with social activities
5a Cut down the amount of time spent on work or other activities
5b Accomplished less than you would like
5c Did work or other activities less carefully than usual
9b Been very nervous
9c Felt so down in the dumps that nothing could cheer you up
9d Felt calm and peaceful
9f Felt downhearted and depressed
9h Been happy

The score of each of the 36 items is collected in CRF (case report form). Then, a SAS (Statistical Analysis System) code (e.g. the one provided by QualityMetric) is used to calculate the eight scales, the two summary measure scores and the standardized summary scores.

The PCS and MCS summary measure scores are computed if at least 50% of the component scales are available. The scale scores are computed if at least 50% of items are available within the corresponding scale. The missing items are imputed by the mean of available items.

Change from baseline (BL) in SF-36 scores (physical component summary score and mental component summary score as well as the eight domains) is then analyzed.

<u>SF-36 V2 Scoring</u>

*General Scoring information:*

Items and scales are scored in 3 steps (as instructed by QualityMetric):

Step 1. Item recoding, for then 10 items that require recoding
Step 2. Computing scale scores by summing across items in the same scale (raw scale scores); and
Step 3. Transforming raw scale scores to a 0-100 scale (transformed scale)
Step 5: Compute Z-Scores
Step 6: Convert Z-score to Norm Based scores for domains
PCS: Compute aggregate PCS score using a specific weighted formula, convert this into a Norm based score
MCS: Compute aggregate MCS score using a specific weighted formula, convert this into a Norm based score

*Item recoding:*

All 36 items should be checked for out-of-range values prior to assigning the final item value. All out-of-range values should be recoded as missing data.

*How to treat missing data*

A scale score is calculated if a respondent answered at least half of the items in a multi-item scale (or half plus one in the case of scales with an odd number of items).
The recommended algorithm substitutes a person-specific estimate for any missing item when the respondent answered at least 50 percent of the items in a scale. A psychometrically sound estimate is the average score, across completed items in the same scale, for that respondent. For example, if a respondent leaves one item in the 5-item mental Health scale blank, substitute the respondent's average score (across the 4 completed mental health items) for that one item. When estimating the respondent's average score, use the respondent's final item values.

*Computing raw scale scores*

After item recoding, including handling of missing data, a raw score is computed for each scale. This score is a simple algebraic sum of responses for all items in that scale. If the respondent answered at least 50%of the items in a multi-items scale, the score can be calculated. If the respondent did not answer at least 50% of the items, the score for that scale should be set to missing.

*Transformation of the scale scores*

The next step involves transforming each raw score to a 0 to 100 scale using the following formula:

Transformed scale = [(actual raw score – lowest possible raw score) / possible raw score range] x 100

This transformation converts the lowest and highest possible scores to zero and 100, respectively.

Table - SF-36 V2 raw scores of eight domains

| Scale Possible | Lowest,Highest possible raw scores | raw score range |
|---|---|---|
| Physical Functioning | 10,30 | 20 |
| Role-Physical | 4,20 | 16 |
| Bodily pain | 2,12 | 10 |
| General health | 5,25 | 20 |
| Vitality | 4,20 | 16 |
| Social Functioning | 2,10 | 8 |

(continued)

| Scale Possible | Lowest,Highest possible raw scores | raw score range |
|---|---|---|
| Role-Emotional | 3,15 | 12 |
| Mental Health | 5,25 | 20 |

FACIT-Fatigue

The Functional Assessment of Chronic Illness Therapy Fatigue scale (FACIT-Fatigue) is used to assess fatigue. The FACIT-Fatigue questionnaire and Scoring & Interpretation Materials are available from FACIT.org (Elmhurst, IL, USA). The FACIT-F questionnaire provides an array of generic and targeted measures with multiple benefits regarding validity, ease of administration, global application, and interpretation, recognized both by the scientific community and the health regulatory agencies.

Briefly, the FACIT-Fatigue is a 13-item questionnaire rated 0 to 4 originally developed to measure fatigue in patients with cancer and is now widely used in rheumatoid arthritis patients to demonstrate good consistency and sensitivity to change. The patient is asked to answer 13 questions rated 0 to 4 (0=not at all, 1 = a little bit, 2 = somewhat, 3 = quite a bit, 4 = very much). The fatigue scale has 13 items, with 52 as the highest possible score. A higher score in the fatigue scale corresponds to a lower level of fatigue and indicates better Quality of Life.

To calculate the FACIT-fatigue score, the response scores on negatively phrased questions are reversed and then the 13 item responses are added. Eleven items with responses have their scores reversed (item score = 4 - response, if the response is not missing), and two items (items 7-8) have their responses unchanged. All items are added so that higher scores correspond to less fatigue. In cases where individual questions are skipped, scores are prorated using the average of other answers in the scale.

FACIT-Fatigue = 13*[sum(reversed items)+su m(items 7-8)]/number of answered items

Sleep questionnaire

Rheumatoid arthritis like other chronic illness is associated with sleep disturbances. Sleep disturbances is linked to pain, mood and disease activity. The effect of sarilumab on pain is assessed on a sleep questionnaire VAS scale ranges from 0 (sleep is not a problem) to 100 (sleep is a major problem).

WPAI

Work Productivity and Activity Impairment (WPAI) is assessed. WPAI outcomes are expressed as impairment percentages, with higher numbers indicating greater impairment and less productivity, ie, worse outcomes, as follows:

- Q1 = currently employed
- Q2 = hours missed due to specified problem (RA)
- Q3 = hours missed other reasons
- Q4 = hours actually worked
- Q5 = degree problem affected productivity while working
- Q6 = degree problem affected regular activities Scores

Scores are expressed in percentages.

- Percent work time missed due to problem (RA): 100*Q2/(Q2+Q4)
- Percent impairment while working due to problem: 10*Q5
- Percent activity impairment due to problem: 10*Q6
- Percent overall work impairment due to problem (RA):

$$1\ 00*Q2/(Q2+Q4)+100*[(1-Q2/(Q2+Q4))*(Q5/10)]$$

When deriving the 4 WPAI scores, missing data is handled using the following rules:

- Percent work time missed due to problem (RA): The score is missing if either Q2 or Q4 is missing.

- Percent impairment while working due to problem: The score is missing if Q5 is missing.
- Percent activity impairment due to problems: The score is missing if Q6 is missing.
- Percent overall work impairment due to problem (RA): The score is missing if either percent work time missed due to problem (RA) is missing or percent impairment while working due to problem is missing.

*Sarilumab*

The invention relates to methods of improving the Health-Related Quality of Life of a subject suffering from rheumatoid arthritis, comprising administering to the subject an effective amount of sarilumab (SAR153191).

The disclosure provides pharmaceutical compositions and methods of using these compositions for improving the Health-Related Quality of Life of a subject suffering from rheumatoid arthritis.

These compositions include at least sarilumab, and, optionally, at least one additional therapeutic agent such as a disease modifying antirheumatic drug (DMARD). Sarilumab is an antibody that specifically binds human interleukin-6 receptor (hIL-6R). The heavy chain variable region of sarilumab comprises the sequence of SEQ ID NO:1. The light chain variable region of sarilumab comprises the sequence of SEQ ID NO:2.

*DMARDs*

Disease modifying antirheumatic drugs (DMARDs) include inter alia methotrexate, sulfasalazine, hydroxychloroquine and leflunomide. According to the compositions and methods of the disclosure, DMARDs can be administered as follows. Methotrexate can be administered from 10 to 25 mg per week orally or intramuscularly. In another embodiment, methotrexate is administered from 6 to 25 mg/week orally or intramuscularly for patients who are from the Asia-Pacific region or who are descended from people who are from the Asia-Pacific region. The Asia-Pacific region includes

Taiwan, South Korea, Malaysia, Philippines, Thailand and India. In certain embodiments, methotrexate is administered at between 6 and 12, 10 and 15, 15 and 20 and 20 and 25 mg per week. In other embodiments, methotrexate is administered at 6, 7, 8, 9, 10, 11, 12, 13, 14, 15, 16, 17, 18, 19, 20, 21, 22, 23, 24 or 25 mg per week. Leflunomide can be administered from 10 to 20 mg orally daily. In certain embodiments, leflunomide can be administered at between 10 and 12, 12 and 15, 15 and 17 and 18 and 20 mg per day. In other embodiments, leflunomide is administered at 10, 11, 12, 13, 14, 15, 16, 17, 18, 19 or 20 mg per day. Sulfasalazine can be administered from 1000 to 3000 mg orally daily. In certain embodiments, sulfasalazine can be administered at between 1000 and 1400, 1400 and 1800, 1800 and 2200, 2200 and 2600, and 2600 and 3000 mg per day. In other embodiments, sulfasalazine is administered at 1000, 1100, 1200, 1300, 1400, 1500, 1600, 1700, 1800, 1900, 2000, 2100, 2200, 2300, 2400, 2500, 2600, 2700, 2800, 2900 or 3000 mg per day. Hydroxychloroquine can be administered from 200 to 400 mg orally daily. In certain embodiments, hydroxychloroquine can be administered at between 200 and 240, 240 and 280, 280 and 320, 320 and 360 and 360 and 400 per day. In other embodiments, hydroxychloroquine can be administered at 200, 210, 220, 230, 240, 250, 260, 270, 280, 290, 300, 310, 320, 330, 340, 350, 360, 370, 380, 390 or 400 mg per day.

*Methods of Administration and Formulations*

The methods described herein comprise administering an effective amount of sarilumab, and, optionally, a DMARD to a patient. As used herein, the phrase "effective amount" means a dose of the antibody that results in a detectable improvement of the Health-Related Quality of Life of a subject suffering from rheumatoid arthritis.

For example, a dose of sarilumab which causes an improvement in any of the following Health-Related Quality of Life scores is deemed an "effective amount":

- the Short Form-36 Physical Component Summary (SF-36 PCS),
- the Short Form-36 Mental Component Summary (SF-36 MCS), - the eight domains of the SF-36:

    ◦ the Short Form-36 Physical Functioning (SF-36 PF),
    ◦ a Short Form-36 Role Physical (SF-36 RP),
    o a Short Form-36 Body Pain (SF-36 BP),
    ◦ a Short Form-36 General Health (SF-36 GH),
    ◦ a Short Form-36 Vitality (SF-36 VT),
    ◦ a Short Form-36 Social Functioning (SF-36 SF),
    ◦ a Short Form-36 Role Emotional (SF-36 RE),
    o a Short Form-36 Mental Health (SF-36 MH),

- a Functional Assessment of Chronic Illness Therapy-Fatigue (FACIT-F). In accordance with the methods of the present invention, an effective amount of sarilumab that is administered to the patient will vary depending upon the age and the size (e.g., body weight or body surface area) of the patient as well as the route of administration and other factors well known to those of ordinary skill in the art. In certain embodiments, the dose of sarilumab administered to the patient is from about 10 mg to about 500 mg. For example, the present invention includes methods wherein about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg, about 120 mg, about 125 mg, about 130 mg, about 135 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200, about 205 mg, about 210 mg, about 215 mg, about 220 mg, about 225 mg, about 230 mg, about 235 mg, about 240 mg, about 245 mg, about 250 mg, about 255 mg, about 260 mg, about 265 mg, about 270 mg, about 275 mg, about 280 mg, about 285 mg, about 290 mg, about 295 mg, about 300, about 325 mg, about 350 mg, about 375 mg, about 400 mg, about 425 mg, about 450 mg, about 475 mg, about 500 mg, or more of sarilumab is administered to the patient per week.

In one embodiment, sarilumab is administered at 100-150 mg per week. In another embodiment, sarilumab is administered at 100-200 mg per ever two weeks. In other embodiments, sarilumab is administered at about 100 or about 150 mg per week. In other embodiments, sarilumab is administered at about 100, 150 or 200 mg per every two weeks.

The amount of sarilumab that is administered to the patient may be expressed in terms of milligrams of antibody per kilogram of patient body weight (*i.e.*, mg/kg). For example, the methods of the present invention include administering sarilumab to a patient at a daily dose of about 0.01 to about 100 mg/kg, about 0.1 to about 50 mg/kg, or about 1 to about 10 mg/kg of patient body weight.

The methods of the present invention include administering multiple doses of sarilumab to a patient over a specified time course. For example, sarilumab can be administered about 1 to 5 times per day, about 1 to 5 times per week, about 1 to 5 times per month or about 1 to 5 times per year. In certain embodiments, the methods of the invention include administering a first dose of sarilumab to a patient at a first time point, followed by administering at least a second dose of sarilumab to the patient at a second time point. The first and second doses, in certain embodiments, may contain the same amount of sarilumab. For instance, the first and second doses may each contain about 10 mg to about 500 mg, about 20 mg to about 300 mg, about 100 mg to about 200 mg, or about 100 mg to about 150 mg of the antibody. The time between the first and second doses may be from about a few hours to several weeks. For example, the second time point (*i.e.,* the time when the second dose is administered) can be from about 1 hour to about 7 weeks after the first time point (*i.e.,* the time when the first dose is administered). According to certain exemplary embodiments of the present invention, the second time point can be about 1 hour, about 4 hours, about 6 hours, about 8 hours, about 10 hours, about 12 hours, about 24 hours, about 2 days, about 3 days, about 4 days, about 5 days, about 6 days, about 7 days, about 2 weeks, about 4 weeks, about 6 weeks, about 8 weeks, about 10 weeks, about 12 weeks, about 14 weeks or longer after the first time point. In certain embodiments, the second time point is about 1 week or about 2 weeks. Third and subsequent doses may be similarly administered throughout the course of treatment of the patient.

The invention provides methods of using compositions comprising sarilumab and, optionally, one or more additional therapeutic agents, e.g., DMARDs. The compositions of the invention will be administered with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTINTM), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

The dose may vary depending upon the age and the weight of a subject to be administered, target disease, conditions, route of administration, and the like. Various delivery systems are known and can be used to administer the pharmaceutical composition of the invention, e.g., encapsulation in liposomes, microparticles, microcapsules, receptor mediated endocytosis (see, *e.g.,* Wu et al. (1987) J. Biol. Chem. 262:4429-4432). Methods of introduction include, but are not limited to, i ntradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (*e.g.,* oral mucosa, rectal and intestinal mucosa, *etc.*) and may be administered together with other biologically active agents. Administration can be systemic or local. Sarilumab can be administered subcutaneously. The DMARD can be administered orally or intramuscularly.

The pharmaceutical composition can also be delivered in a vesicle, in particular a liposome (see Langer (1990) Science 249:1527-1533). In certain situations, the pharmaceutical composition can be delivered in a controlled release system, for example, with the use of a pump or polymeric materials. In another embodiment, a controlled release system can be placed in proximity of the composition's target, thus requiring only a fraction of the systemic dose.

The injectable preparations may include dosage forms for intravenous, subcutaneous, intracutaneous and intramuscular injections, local injection, drip infusions, *etc.* These injectable preparations may be prepared by methods publicly known. For example, the injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, *etc.,* which may be used in combination with an appropriate solubilizing agent such as an alcohol (*e.g.,* ethanol), a polyalcohol (*e.g.,* propylene glycol, polyethylene glycol), a nonionic surfactant [e.g., polysorbate 80, HCO-50 (polyoxyethylene (50 mol) adduct of hydrogenated castor oil)], *etc.* As the oily medium, there are employed, *e.g.,* sesame oil, soybean oil, *etc.,* which may be used in combination with a solubilizing agent such as benzyl benzoate, benzyl alcohol, *etc.* The injection thus prepared can be filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, *etc.* The amount of the DMARD contained is generally about 5 to 3000 mg per dosage form in an oral unit dose depending on the specific DMARD used. The amount of the sarilumab contained is generally about 100 to 200 mg per subcutaneous dosage form.

In a particular embodiment, sarilumab is administered subcutaneously, as an aqueous buffered solution at pH 6.0 containing:

- 25 mM histidine
- 50 mM arginine
- 0.2% (w/v) polysorbate 20, and
- 5% (w/v) sucrose
- between 100 mg/mL and 200 mg/mL sarilumab.

In another particular embodiment, sarilumab is administered subcutaneously, as an aqueous buffered solution at pH 6.0 containing:

- 25 mM histidine
- 50 mM arginine
- 0.2% (w/v) polysorbate 20, and
- 5% (w/v) sucrose
- between 131 and 132 mg/mL of sarilumab, more particularly 131 .6mg/mL of sarilumab.

In still another particular embodiment, sarilumab is administered subcutaneously, as an aqueous buffered solution at pH 6.0 containing:

- 25 mM histidine
- 50 mM arginine
- 0.2% (w/v) polysorbate 20, and
- 5% (w/v) sucrose
- 1 75mg/mL of sarilumab.

In accordance with the methods disclosed herein, sarilumab (or pharmaceutical formulation comprising the antibody) can be administered to the patient using any acceptable device or mechanism. For example, the administration can be accomplished using a syringe and needle or with a reusable pen and/or autoinjector delivery device. The methods of the present invention include the use of numerous reusable pen and/or autoinjector delivery devices to administer an antibody (or pharmaceutical formulation comprising the antibody). Examples of such devices include, but are not limited to AUTOPENTM (Owen Mumford, Inc., Woodstock, UK), DISETRONICTM pen (Disetronic Medical Systems, Bergdorf, Switzerland), HUMALOG MIX 75/25TM pen, HUMALOGTM pen, HUMALIN 70/30TM pen (Eli Lilly and Co., Indianapolis, IN), NOVOPENTM I, II and III (Novo Nordisk, Copenhagen, Denmark), NOVOPEN JUNIORTM (Novo Nordisk, Copenhagen, Denmark), BDTM pen (Becton Dickinson, Franklin Lakes, NJ), OPTIPENTM, OPTIPEN PROTM, OPTIPEN STARLETTM, and OPTICLIKTM (sanofi-aventis, Frankfurt, Germany), to name only a few. Examples of disposable pen and/or autoinjector delivery devices having applications in subcutaneous delivery of a pharmaceutical composition of the present invention include, but are not limited to the SOLOSTARTM pen (sanofi-aventis), the FLEXPENTM (Novo Nordisk), and the KWIKPENTM (Eli Lilly), the SURECLICKTM Autoinjector (Amgen, Thousand Oaks, CA), the PENLETTM (Haselmeier, Stuttgart, Germany), the EPIPEN (Dey, L.P.), and the HUMIRATM Pen (Abbott Labs, Abbott Park, IL), to name only a few.

The use of a microinfusor to deliver sarilumab (or pharmaceutical formulation comprising the antibody) to a patient is also contemplated herein. As used herein, the term "microinfusor" means a subcutaneous delivery device designed to slowly administer large volumes (e.g., up to about 2.5 mL or more) of a therapeutic formulation over a prolonged period of time (e.g., about 10, 15, 20, 25, 30 or more minutes). See, *e.g.,* U.S. 6,629,949; US 6,659,982; and Meehan et al., J. Controlled Release 46:107-116 (1996). Microinfusors are particularly useful for the delivery of large doses of therapeutic proteins contained within high concentration (e.g., about 100, 125, 150, 175, 200 or more mg/mL) and/or viscous solutions.

*Combined administration*

The present disclosure provides methods of improving the Health-Related Quality of Life of a subject suffering from rheumatoid arthritis, which comprise administering to the subject sarilumab. In certain embodiments, the anti-hIL-6 antibody is administered as a "monotherapy" or single therapeutic agent. In alternative embodiments, the anti-hIL-6 antibody is administered in combination with at least one additional therapeutic agent. Examples of additional therapeutic agents which can be administered in combination with sarilumab in the practice of the methods of the present invention include, but are not limited to DMARDs, and any other compound known to treat, prevent, or ameliorate rheumatoid arthritis in a human subject. Specific, non-limiting examples of additional therapeutic agents that may be administered in combination with sarilumab in the context of a method of the present invention include, but are not limited to methotrexate, sulfasalazine, hydroxychloroquine and leflunomide. In the present methods, the additional therapeutic agent(s) can be administered concurrently or sequentially with sarilumab. For example, for concurrent administration, a pharmaceutical formulation can be made which contains both sarilumab and at least one additional therapeutic agent. The amount of the additional therapeutic agent that is administered in combination with sarilumab in the practice of the methods of the present invention can be easily determined using routine methods known and readily available in the art.

The disclosure of the invention provides for pharmaceutical compositions comprising any of the following:

A composition comprising between 100 and 150 mg of sarilumab (SAR153191) and 10-25 mg of methotrexate.
A composition comprising between 100 and 200 mg of sarilumab (SAR153191) and 10-25 mg of methotrexate.
A composition comprising between 100 and 150 mg of sarilumab (SAR153191) and 6-25 mg of methotrexate.
A composition comprising between 100 and 200 mg of sarilumab (SAR153191) and 6-25 mg of methotrexate.
A composition comprising between 100 and 150 mg of sarilumab (SAR153191) and 10-20 mg of leflunomide.
A composition comprising between 100 and 200 mg of sarilumab (SAR153191) and 10-20 mg of leflunomide.
A composition comprising between 100 and 150 mg of sarilumab (SAR153191) and 1000-3000 mg of sulfasalazine.
A composition comprising between 100 and 200 mg of sarilumab (SAR153191) and 1000-3000 mg of sulfasalazine.
A composition comprising between 100 and 150 mg of sarilumab (SAR153191) and 200-400 mg of hydroxychloroquine.
A composition comprising between 100 and 200 mg of sarilumab (SAR153191) and 200-400 mg of hydroxychloroquine.

The disclosure of the invention provides for methods of improving the Health-Related Quality of Life of a subject suffering from rheumatoid arthritis comprising any of the following:

A method comprising administering 150 mg or 200 mg of sarilumab (SAR153191) every two weeks and 10-25 mg of methotrexate per week to a subject in need thereof.
A method comprising administering 150 mg or 200 mg of sarilumab (SAR153191) every two weeks and 6-25 mg of methotrexate per week to a subject in need thereof.
A method comprising administering 150 mg or 200 mg of sarilumab (SAR153191) every two weeks and 10-20 mg of leflunomide per day to a subject in need thereof.
A method comprising administering 150 mg or 200 mg of sarilumab (SAR153191) every two weeks and 1000-3000 mg of sulfasalazine per day to a subject in need thereof.
A method comprising administering between 150 mg or 200 mg of sarilumab (SAR153191) every two weeks and 200-400 mg of hydroxychloroquine per day to a subject in need thereof.

*Patient Population*

In certain embodiments, the methods and compositions described herein are administered to specific patient populations. These populations include patients that have previously been treated for rheumatoid arthritis with treatment regimens other than the combination of sarilumab and one or more DMARDs. In other embodiments, sarilumab is administered to a patient who has previously been ineffectively treated with a DMARD and an anti-hIL-6R antibody other than sarilumab. These treatment regimens include anti-TNF-a therapy, e.g., biologic anti-TNF-a treatment regimens. Biologic anti-TNF-a antagonists include etanercept, infliximab, adalimumab, golimumab and certolizumab pegol. These treatment regimens also include DMARD therapy in the absence of anti-hIL-6R antibody.

DMARDs used in this therapy include methotrexate, sulfasalazine, hydroxychloroquine and leflunomide. The DMARDs may be administered alone or in combination with another therapy that is not an anti-hIL-6R antibody, e.g., Sarilumab. In a specific embodiment, the previous treatment regimen was methotrexate. In another embodiment, treatment with methotrexate is maintained in patient treated with sarilumab. In certain embodiments, the patient has previously been administered both anti-TNF-a and DMARD therapies. The therapies may be performed sequentially in any order or simultaneously. In certain embodiments, these therapies have been received by the patient within 2 years prior to receiving the combination of sarilumab and one or more DMARDs. In other embodiments, these therapies have been received within 1, 2, 3, 4, 5, 6, 7, 8, 9 or 10 years prior to receiving the combination of sarilumab and one or more DMARDs.

In certain embodiments, the methods and compositions described herein are administered to specific patient populations that have received one or more of the treatment regimens described above wherein these treatments have not been effective. As used herein, a treatment has not been effective when the treatment (e.g., a dose of anti-TNF-a and/or a DMARD) does not result in a detectable improvement in one or more symptoms associated with rheumatoid arthritis or which does not cause a biological effect (e.g., a decrease in the level of a particular biomarker) that is correlated with the underlying pathologic mechanism(s) giving rise to the condition or symptom(s) of rheumatoid arthritis. For example, a treatment which does not cause an improvement in any of the following symptoms or conditions is deemed ineffective: chronic disease anemia, fever, depression, fatigue, rheumatoid nodules, vasculitis, neuropathy, scleritis, pericarditis, Felty's syndrome and/or joint destruction.

A detectable improvement can also be detected using the American College of Rheumatism (ACR) rheumatoid arthritis classification criteria. For example a 20% (ACR20), 50% (ACR50) or 70% (ACR70) improvement from baseline can be used to show detectable improvement. Conversely, the ACR classification criteria may be used to select patients who have previously been ineffectively treated prior to treatment with anti-IL6R therapy. For example, a patient may be ineffectively treated if they fail to exhibit at least a 10% detectable improvement (e.g., a 10%, 20%, 50% or 70% improvement) according to ACR criteria.

In other embodiments, the disease activity score (DAS28) can be used to show detectable improvement or, conversely, ineffective treatment. DAS28 is a composite score of tender joints count based on 28 joints, a swollen joints count based on 28 joints, a general health assessment and a marker of inflammation which can be assessed by measuring C-reactive protein (CRP) levels. The disease response can be presented using the European League against Rheumatism (EULAR) response criteria. A good response by this criteria is an improvement of greater than 1.2 in DAS28 score with a present score of greater than or equal to 3.2. A moderate response is an improvement of greater than 0.6 but less than or equal to 1.2 in DAS28 score and a present score of greater than 3.2. Non-response is an improvement of less than 0.6 in DAS28 score and a present score of greater than 5.1. DAS28 remission is a DAS28 score of less than 2.6. A detectable improvement can also be shown by measuring an improvement in any of the components of the DAS28 score.

In other exemplary embodiments, a treatment has not been effective when a patient still presents an "active disease" after treatment. For example, patients present an "active disease" when they exhibit at least 8 of 68 tender joints and 6 of 66 swollen joints, and/or high sensitivity C-reactive protein (hs-CRP) >10 mg/L (>1.0 mg/dL). In a specific embodiment, sarilumab is administered to a patient who has previously been ineffectively treated with methotrexate (MTX). In such an example, patients may have received continuous treatment with MTX 10 to 25 mg/week (or per local labeling requirements if the dose range differs) for at least 12 weeks and on a stable dose of MTX for a minimum of 8 weeks and still present a moderate-to-severely active RA, defined as: (i) at least 8 of 68 tender joints and 6 of 66 swollen joints, and (ii) high sensitivity C-reactive protein (hs-CRP) >10 mg/L (>1.0 mg/dL).

In one embodiment, the patient population comprises subjects that have not received a biologic agent within the last three months. In certain embodiments, a biologic agent is a protein. According to other embodiments, the protein is an antibody or a fragment thereof. According to other embodiments, the protein is a cytokine or a fragment or derivative thereof. In other embodiments, the protein is recombinant. In other embodiments, the biologic agent is a vaccine, blood, blood component, allergenic, somatic cell, gene therapy or biological tissue. Biologics include blood factors, thrombolytic agents, hormones, hematopoietic growth factors, interferons, interleukin based products, vaccines and monoclonal antibodies. In certain embodiments, the biologic agent is abatacept. In other embodiments, the biologic agent is adalimumab.

In other embodiments, biologic agents non-exhaustively include: TNFα blockers such as infliximab (Remicade®), adalimumab (Humira®), golimumab (Simponi®), etanercept (Enbrel®), certolizumab (Cimzia®); IL-1 blockers such as anakinra (Kineret®), anti-CD20 antibodies such as rituximab (Rituxan®), T cell costimulation blocker such as abatacept (Orencia®), and anti-IL6 antibodies such as sirukumab, clazakizumab or olokizu mab.

According to other embodiments, the biologic is a biologic medical product. A biologic medical product is a medical product that is manufactured or extracted from biological sources. In certain embodiments, biologics are defined as excluding biological products that are consumed by animals for nutrition. In these embodiments, biologics must have a specific therapeutic consequence to the subject population beyond nutrition.

In another embodiment, the patient population comprises subjects that have received treatment for methotrexate for at least 6 weeks. In other embodiments, the subjects have received methotrexate for at least 1, 2, 3, 4, 5, 6, 7, 8, 9, 10, 11, 12, 13, 14, 15, 16, 17, 18, 19, 20, 21, 22, 23, 24, 25 or 26 weeks. In more specific embodiments, the subjects have received methotrexate but have not received other DMARDs for at least 6 weeks. In other embodiments, the subjects have not received a DMARD other than methotrexate for at least 1, 2, 3, 4, 5, 6, 7, 8, 9, 10, 11, 12, 13, 14, 15, 16, 17, 18, 19, 20, 21, 22, 23, 24, 25 or 26 weeks. DMARDs other than methotrexate include leflunomide, sulfasalazine and hydroxychloroquine.

In another embodiment, the patient population comprises subjects that are not suffering from an autoimmune disease other than rheumatoid arthritis. In certain embodiments, the autoimmune disease other than arthritis is one or more pathologies selected from the group consisting of Acute disseminated encephalomyelitis (ADEM), Addison's disease, Agammaglobulinemia, Alopecia areata, Amyotrophic lateral sclerosis (Also Lou Gehrig's disease; Motor Neuron Disease), Ankylosing Spondylitis, Antiphospholipid syndrome, Antisynthetase syndrome, Atopic allergy, Atopic dermatitis, Autoimmune aplastic anemia, Autoimmune cardiomyopathy, Autoimmune enteropathy, Autoimmune hemolytic anemia, Autoimmune hepatitis, Autoimmune inner ear disease, Autoimmune lymphoproliferative, Autoimmune peripheral neuropathy, Autoimmune pancreatitis, Autoimmune polyendocrine syndrome, Autoimmune progesterone dermatitis, Autoimmune thrombocytopenic purpura, Autoimmune urticaria, Autoimmune uveitis, Balo disease/Balo concentric sclerosis, Behçet's disease, Berger's disease, Bickerstaff's encephalitis, Blau syndrome, Bullous pemphigoid, Cancer, Castleman's disease, Celiac disease, Chagas disease, Chronic inflammatory demyelinating polyneuropathy, Chronic recurrent multifocal osteomyelitis, Chronic obstructive pulmonary disease, Churg-Strauss syndrome, Cicatricial pemphigoid, Cogan syndrome, Cold agglutinin disease, Complement component 2 deficiency, Contact dermatitis, Cranial arteritis, Crohn's disease, Cushing's Syndrome, Cutaneous leu kocytoclastic angiitis, Dego's disease, Dercu m's disease, Dermatitis herpetiformis, Dermatomyositis, Diabetes mellitus type 1, Diffuse cutaneous systemic sclerosis, Dressler's syndrome, Drug-induced lupus, Discoid lupus erythematosus, Eczema, Endometriosis, Enthesitis-related arthritis, Eosinophilic fasciitis, Eosinophilic, Eosinophilic pneumonia, Epidermolysis bullosa acquisita, Erythema nodosum, Erythroblastosis fetalis, Essential mixed cryoglobulinemia, Evan's syndrome, Fibrodysplasia ossificans prog ressiva, Fibrosing alveolitis, Gastritis, Gastrointestinal pemphigoid, Glomerulonephritis, Goodpasture's syndrome, Graves' disease, Guillain-Barre syndrome (GBS), Hashimoto's encephalopathy, Hashimoto's thyroiditis, Henoch-Schonlein purpura, Herpes gestationis, Hidradenitis suppurativa, Hughes-Stovin syndrome, Hypogammaglobulinemia, Idiopathic inflammatory demyelinating diseases, Idiopathic pulmonary fibrosis, Idiopathic thrombocytopenic purpura, IgA nephropathy, Inclusion body myositis, Chronic inflammatory demyelinating polyneuropathy, Interstitial cystitis, Juvenile idiopathic arthritis, Kawasaki's disease, Lambert-Eaton myasthenic syndrome, Leukocytoclastic vasculitis, Lichen planus, Lichen sclerosus, Linear IgA disease (LAD), Lupoid hepatitis, Lupus erythematosus, Majeed syndrome, Ménière's disease, Microscopic polyangiitis, Miller-Fisher syndrome, Mixed connective tissue disease, Morphea, Mucha-Habermann disease, Multiple sclerosis, Myasthenia gravis, Microscopic colitis, Myositis, Narcolepsy, Neuromyelitis optica, Neuromyotonia, Occular cicatricial pemphigoid, Opsoclonus myoclonus syndrome, Ord's thyroiditis, Palindromic rheumatism, PANDAS (pediatric autoi mmune neuropsychiatric disorders associated with streptococcus), Paraneoplastic cerebellar degeneration, Paroxysmal nocturnal hemoglobinuria, Parry Romberg syndrome, Parsonage-Turner syndrome, Pars planitis, Pemphigus vulgaris, Pernicious anaemia, Perivenous encephalomyelitis, POEMS syndrome, Polyarteritis nodosa, Polymyalgia rheumatica, Polymyositis, Primary biliary cirrhosis, Primary sclerosing cholangitis, Progressive inflammatory neuropathy, Psoriasis, Psoriatic arthritis, Pyoderma gangrenosum, Pure red cell aplasia, Rasmussen's encephalitis, Raynaud phenomenon, Relapsing polychondritis, Reiter's syndrome, Restless leg syndrome, Retroperitoneal fibrosis, Rheumatic fever, Sarcoidosis, Schizophrenia, Schmidt syndrome, Schnitzler syndrome, Scleritis, Scleroderma, Serum Sickness, Sjögren's syndrome, Spondyloarth ropathy, Still's disease, Stiff person syndrome, Subacute bacterial endocarditis, Susac's syndrome, Sweet's syndrome, Sydenham chorea, Sympathetic ophthalmia, Systemic lupus erythematosus, Takayasu's arteritis, Temporal arteritis, Thrombocytopenia, Tolosa-Hunt syndrome, Transverse myelitis, Ulcerative colitis, Undifferentiated connective tissue disease, Undifferentiated spondyloarthropathy, Urticarial vasculitis, Vasculitis, Vitiligo and Wegener's granulomatosis

In another embodiment, the patient population comprises subjects that have not received parenteral or intra-articular administration of glucocorticoids for four weeks. In other embodiments, the subjects have not received parenteral or intra-articular administration of glucocorticoids for at least 1, 2, 3, 4, 5, 6, 7, 8, 9, 10, 11, 12, 13, 14, 15, 16, 17, 18, 19, 20, 21, 22, 23, 24, 25 or 26 weeks. In certain embodiments, the glucocorticoids include one or more selected from the group consisting of cortisol, cortisone, prednisone, prednisolone, methylprednisolone, dexamethasone, betamethasone, fludrocortisone, deoxycorticosterone acetate and aldosterone.

The methods according to the invention include administering to the subject an effective amount of sarilumab alone, or an effective amount of sarilumab and a member of the group consisting of leflunomide, sulfasalazine and hydroxychloroquine. In certain embodiments, the subject was previously ineffectively treated for rheumatoid arthritis by administering a TNF-α antagonist. Specifically, subject could have been treated for at least three months with the TNF-α antagonist or could have been intolerant of the TNF-α antagonist. The TNF-α antagonist could be etanercept, infliximab, adalimumab, golimumab or certolizumab. In other embodiments, the subject was previously ineffectively treated for rheumatoid arthritis by administering methotrexate.

Sarilumab could be administered at between 50 and 150 mg per week or between 100 and 200 mg per two weeks.

In certain specific embodiments, sarilumab and leflunomide are administered to the subject. The leflunomide can be administered orally. The leflunomide can also be administered at between 10 and 20 mg per day to the subject. In other specific embodiments, sarilumab and sulfasalazine are administered to the subject. The sulfasalazine can be administered orally. The sulfasalazine can also be administered at between 1000 to 3000 mg per day to the subject.

In other specific embodiments, sarilumab and hydroxychloroquine are administered to the subject. The hydroxychloroquine can be administered orally. The hydroxychloroquine can also be administered at between 200 to 400 mg per day to the subject.

The present disclosure also provides a method of improving the Health-Related Quality of Life of a subject suffering from rheumatoid arthritis, comprising administering to the subject an effective amount of sarilumab and methotrexate, wherein the subject was previously ineffectively treated for rheumatoid arthritis by administering an anti-TNF-α therapeutic. In certain embodiments, the subject was previously ineffectively treated for rheumatoid arthritis by administering methotrexate. The methotrexate can be administered at between 10 to 25 mg per week to the subject.

In certain embodiments, the subject is a mammal. The mammal can be a human. In certain embodiments, the human is descended from individuals from Asia or the Pacific. Humans descended from individuals from Asia or the Pacific can be administered between 6 and 25 mg per week of methotrexate.

In certain embodiments, the subject was previously ineffectively treated for rheumatoid arthritis by administering a TNF-α antagonist. Specifically, subject could have been treated for at least three months with the TNF-α antagonist or could have been intolerant of the TNF-α antagonist. The TNF-α antagonist could be etanercept, infliximab, adalimumab, golimumab or certolizumab. In other embodiments, the subject was previously ineffectively treated for rheumatoid arthritis by administering methotrexate.

The sarilumab could be administered at between 50 and 150 mg per week or between 100 and 200 mg per two weeks.

The disclosure also provides a pharmaceutical composition comprising an effective amount of sarilumab and a member of the group consisting of leflunomide, sulfasalazine and hydroxychloroquine. The sarilumab could be present at between 50 and 150 mg per dose or between 100 and 200 mg per dose.

In certain specific embodiments, the composition includes sarilumab and leflunomide. The leflunomide can be present in an oral dosage form. The leflunomide can be present in the composition at between 10 and 20 mg per dose.

In other specific embodiments, the composition includes sarilumab and sulfasalazine. The sulfasalazine can be present in an oral dosage form. The sulfasalazine can be present in the composition at between 1000 to 3000 mg per day to the subject.

In other specific embodiments, the composition includes sarilumab and hydroxychloroquine. The hydroxychloroquine can be present in an oral dosage form. The hydroxychloroquine can be present in the composition at between 200 to 400 mg per day to the subject.

In certain embodiments, the disclosure also provides a method of treating rheumatoid arthritis in a subject previously treated by administering methotrexate, leflunomide, sulfasalazine and/or hydroxychloroquine, comprising administering to the subject an effective amount of sarilumab.

In one embodiment, the subject was previously ineffectively treated for rheumatoid arthritis by administering methotrexate, leflunomide, sulfasalazine and/or hydroxychloroquine.

In another embodiment, sarilumab is administered as a monotherapy.

In another embodiment, methotrexate, leflunomide, sulfasalazine and/or hydroxychloroquine is administered together with sarilumab.

In another embodiment, sarilumab and methotrexate are administered together. In one embodiment, methotrexate is administered between 6 to 25 mg per week.

In certain embodiments, sarilumab is administered at between 50 and 150 mg per week. In other embodiments, the sarilumab is administered at between 100 and 200 mg per two weeks. In other embodiments, the sarilumab is administered at 100 mg per two weeks. In other embodiments, the sarilumab is administered at 150 mg per two weeks. In other embodiments, the sarilumab is administered at 200 mg per two weeks. In certain embodiments, the subject was previously ineffectively treated for rheumatoid arthritis by administering a TNF-α antagonist. In one embodiment, the subject has been treated with an anti-TNF-a antagonist for at least 3 months in the last 2 years or the subject was intolerant to at least one TNF-α antagonist. In another embodiment, the TNF-α antagonist is a biologic anti-TNF-a. In another embodiment, the TNF-α antagonist is selected from the group consisting in etanercept, infliximab, adalimumab, golimumab and/or certolizumab pegol.

In other embodiments, the disclosure provides a pharmaceutical composition comprising an effective amount of sarilumab and a member of the group consisting of methotrexate, leflunomide, sulfasalazine and hydroxychloroquine.

In yet other embodiments, the disclosure provides a combination of: a pharmaceutical composition comprising sarilumab, and a pharmaceutical composition comprising methotrexate, leflunomide, sulfasalazine or hydroxychloroquine for sequential or simultaneous use as a medicament.

The present invention further relates to the following items:

1. A method of improving the Health-Related Quality of Life of a subject suffering from rheumatoid arthritis, comprising administering to the subject an effective amount of sarilumab.

2. The method according to item 1, wherein the subject achieves after at least 24 weeks of treatment a change from baseline (BL) in any one or more Health-Related Quality of Life outcomes selected from the group consisting of:

- a Short Form-36 Physical Component Summary (SF-36 PCS) change from BL of at least 2.5,
- a Short Form-36 Mental Component Summary (SF-36 MCS) change from BL of at least 2.5,
- eight domains of the SF-36:

   o a Short Form-36 Physical Functioning (SF-36 PF) change from BL of at least 5,
   o a Short Form-36 Role Physical (SF-36 RP) change from BL of at least 5,
   o a Short Form-36 Body Pain (SF-36 BP) change from BL of at least 5,
   o a Short Form-36 General Health (SF-36 GH) change from BL of at least 5,
   o a Short Form-36 Vitality (SF-36 VT) change from BL of at least 5,
   o a Short Form-36 Social Functioning (SF-36 SF) change from BL of at least 5,
   o a Short Form-36 Role Emotional (SF-36 RE) change from BL of at least 5,
   o a Short Form-36 Mental Health (SF-36 MH) change from BL of at least 5,

- a Functional Assessment of Chronic Illness Therapy-Fatigue (FACIT-F) change from BL of at least 3.

3. The method according to item 1 or 2, wherein the subject achieves after at least 24 weeks of treatment with sarilumab a change from baseline (BL) greater than the change from BL obtained after at least 24 weeks of treatment with placebo in all eight domains of the SF-36, as well as in SF-36 PCS, SF-36 MCS.

4. The method according to item 3, wherein the change from baseline (BL) greater than the change from BL obtained after at least 24 weeks of treatment with placebo in all eight domains of the SF-36, as well as in SF-36 PCS, SF-36 MCS, consists of:

- a SF-36 PCS change from BL of at least 5.3,
- a SF-36 MCS change from BL of at least 4,
- a SF-36 PF change from BL of at least 5,
- a SF-36 RP change from BL of at least 5,
- a SF-36 BP change from BL of at least 6.6,
- a SF-36 GH change from BL of at least 5,
- a SF-36 VT change from BL of at least 5.5,
- a SF-36 SF change from BL of at least 5,
- a SF-36 RE change from BL of at least 5,
- a SF-36 MH change from BL of at least 5,
- a FACIT-F change from BL of at least 6.5.

5. The method according to item 1 or 2, wherein the subject achieves after at least 24 weeks of treatment a change from baseline (BL) in any one or more Health-Related Quality of Life outcomes selected from the group consisting of:

- a SF-36 PCS change from BL of at least 5.3,
- a SF-36 MCS change from BL of at least 4,
- a SF-36 PF change from BL of at least 5,
- a SF-36 RP change from BL of at least 5,
- a SF-36 BP change from BL of at least 6.6,

- a SF-36 GH change from BL of at least 5,
- a SF-36 VT change from BL of at least 5.5,
- a SF-36 SF change from BL of at least 5,
- a SF-36 RE change from BL of at least 5,
- a SF-36 MH change from BL of at least 5,
- a FACIT-F change from BL of at least 6.5.

6. The method according to any one of items 1 to 5, wherein the subject achieves after at least 24 weeks of treatment a score in any one or more Health-Related Quality of Life outcomes selected from the group consisting of:

- a SF-36 PCS change from baseline (BL) of at least 8,
- a SF-36 MCS change from BL of at least 5,
- a SF-36 PF change from BL of at least 7,
- a SF-36 RP change from BL of at least 7,
- a SF-36 BP change from BL of at least 10,
- a SF-36 GH change from BL of at least 6,
- a SF-36 VT change from BL of at least 7,
- a SF-36 SF change from BL of at least 7,
- a SF-36 RE change from BL of at least 6,
- a SF-36 MH change from BL of at least 5,
- a FACIT-F change from BL of at least 9.

7. The method of any one of items 1 to 6, wherein the subject was previously ineffectively treated for rheumatoid arthritis by administering at least one disease-modifying anti-rheumatic drug (DMARD).

8. The method of item 7, wherein the at least one DMARD is selected from the group consisting of methotrexate, leflunomide, sulfasalazine, hyd roxychloroquine, abatacept, rituximab, tocilizu mab, adalimumab, certolizumab pegol, etanercept, golimumab and infliximab.

9. The method of item 7, wherein the at least one DMARD is selected from the group consisting of methotrexate, leflunomide, sulfasalazine and hyd roxychloroquine.

10. The method of item 7, wherein the at least one DMARD is selected from the group consisting of adalimumab, certolizumab pegol, etanercept, golimumab and infliximab.

11. The method of any of items 1 to 10, wherein the method comprises administering to the subject an effective amount of sarilumab and an effective amount of methotrexate, leflunomide, sulfasalazine and/or hydroxychloroquine.

12. The method of any of items 1 to 11, wherein the method comprises administering to the subject an effective amount of sarilumab and an effective amount of methotrexate.

13. The method of item 12, wherein methotrexate is administered between 6 to 25 mg per week.

14. The method of any of item 1 to 13, wherein sarilumab is administered subcutaneously.

15. The method of any of item 1 to 14, wherein sarilumab is administered at 150 mg
per two weeks or at 200 mg per two weeks.

16. Sarilumab for use in a method of improving the Health-Related Quality of Life of a subject suffering from rheumatoid arthritis.

17. Sarilumab for use according to item 16, wherein the subject achieves after at least 24 weeks of treatment a change from baseline (BL) in any one or more Health-Related Quality of Life outcomes selected from the group consisting of:

- a Short Form-36 Physical Component Summary (SF-36 PCS) change from BL of at least 2.5,
- a Short Form-36 Mental Component Summary (SF-36 MCS) change from BL of at least 2.5,
- eight domains of the SF-36:

○ a Short Form-36 Physical Functioning (SF-36 PF) change from BL of at least 5,
○ a Short Form-36 Role Physical (SF-36 RP) change from BL of at least 5,
○ a Short Form-36 Body Pain (SF-36 BP) change from BL of at least 5,
○ a Short Form-36 General Health (SF-36 GH) change from BL of at least 5,
○ a Short Form-36 Vitality (SF-36 VT) change from BL of at least 5,
○ a Short Form-36 Social Functioning (SF-36 SF) change from BL of at least 5,
○ a Short Form-36 Role Emotional (SF-36 RE) change from BL of at least 5,
○ a Short Form-36 Mental Health (SF-36 MH) change from BL of at least 5,

- a Functional Assessment of Chronic Illness Therapy-Fatigue (FACIT-F) change from BL of at least 3.

18. Sarilumab for use according to item 16 or 17, wherein the subject achieves after at least 24 weeks of treatment with sarilumab a change from baseline (BL) greater than the change from BL obtained after at least 24 weeks of treatment with placebo in all eight domains of the SF-36, as well as in SF-36 PCS, SF-36 MCS.

19. Sarilumab for use according to item 18, wherein the change from baseline (BL)
greater than the change from BL obtained after at least 24 weeks of treatment with placebo in all eight domains of the SF-36, as well as in SF-36 PCS, SF-36 MCS, consists of:

- a SF-36 PCS change from BL of at least 5.3,
- a SF-36 MCS change from BL of at least 4,
- a SF-36 PF change from BL of at least 5,
- a SF-36 RP change from BL of at least 5,
- a SF-36 BP change from BL of at least 6.6,
- a SF-36 GH change from BL of at least 5,
- a SF-36 VT change from BL of at least 5.5,
- a SF-36 SF change from BL of at least 5,
- a SF-36 RE change from BL of at least 5,
- a SF-36 MH change from BL of at least 5,
- a FACIT-F change from BL of at least 6.5.

20. Sarilumab for use according to item 16 or item 17, wherein the subject achieves after at least 24 weeks of treatment a change from baseline (BL) in any one or more Health-Related Quality of Life outcomes selected from the group consisting of:

- a SF-36 PCS change from BL of at least 5.3,
- a SF-36 MCS change from BL of at least 4,
- a SF-36 PF change from BL of at least 5,
- a SF-36 RP change from BL of at least 5,
- a SF-36 BP change from BL of at least 6.6,
- a SF-36 GH change from BL of at least 5,
- a SF-36 VT change from BL of at least 5.5,
- a SF-36 SF change from BL of at least 5,
- a SF-36 RE change from BL of at least 5,
- a SF-36 MH change from BL of at least 5,
- a FACIT-F change from BL of at least 6.5.

21. Sarilumab for use according to any of item 16 to 20, wherein the subject achieves after at least 24 weeks of treatment a score in any one or more Health-Related Quality of Life outcomes selected from the group consisting of:

- a SF-36 PCS change from baseline (BL) of at least 8,
- a SF-36 MCS change from BL of at least 5,
- a SF-36 PF change from BL of at least 7,
- a SF-36 RP change from BL of at least 7,
- a SF-36 BP change from BL of at least 10,
- a SF-36 GH change from BL of at least 6,
- a SF-36 VT change from BL of at least 7,
- a SF-36 SF change from BL of at least 7,

- a SF-36 RE change from BL of at least 6,
- a SF-36 MH change from BL of at least 5,
- a FACIT-F change from BL of at least 9.

**EXAMPLE:**

**Impact of Sarilumab on Health Related Quality of Life [HRQoL], Fatigue, and Sleep in Rheumatoid Arthritis Patients at Week 24 - Results of a Phase 3, Randomized, Double-Blind, Placebo-Controlled Multi-center Study.**

Sarilumab, a human monoclonal antibody directed against IL-6 receptor, demonstrated efficacy in phase 3 of the SARIL-RA-MOBILITY study in adults with active, moderate-to-severe RA with inadequate responses to methotrexate (MTX). Both doses of sarilumab (150mg & 200 mg every other week [q2w]) met clinical, radiographic, functional, and safety endpoints.

The objective of the present additional study was to evaluate the impact of sarilumab on Health Related Quality of Life (HRQoL), fatigue, and sleep, all of which were pre-defined secondary endpoints based on mean changes from baseline [BL] at week 24 among patients who had a patient reported outcome (PRO) measured at that time point. In addition, Work productivity impairment was assessed at week 12.

Methods:

1,282 patients were randomized to receive placebo (PBO), sarilumab 150 mg q2w or 200 mg q2w (1:1:1) plus background MTX; the intent to treat population included 1,197. The Short Form-36 [SF-36], Functional Assessment of Chronic Illness Therapy-Fatigue (FACIT-F), Sleep Visual Analog Scale (VAS) and Work Productivity and Activity Impairment [WPAI] questionnaires were assessed at BL, weeks 12 [WPAI only], week 24 and 52.

Results:

Statistically significant improvements versus PBO in SF-36 Physical Component Summary (PCS) and Mental Component Summary (MCS), all 8 domains of SF-36, FACIT-F and Sleep VAS were reported by patients receiving sarilumab 150mg and 200mg at week 24, which well-exceeded the minimum clinically important difference (MCID) in all SF-36 [the MCID for PCS and MCS is a change from BL of at least 2.5; MCID for the 8 domains is a change from BL of at least 5.0, MCID for the FACIT-F is a change from BL of at least 3.0, and MCID for the Sleep VAS is a change from BL of at least 4.1] scores in both active treatment groups (Table 1, Bolded). Statistically significant improvements in WPAI '% overall work impairment due to RA' scores were reported for 150 mg group at week 12. Improvements evident at week 24 were sustained through week 52 [Data not shown].

Conclusions: In this Phase 3 trial, patients with active RA receiving both doses of sarilumab reported clinically meaningful and statistically significant improvements versus placebo in all HRQoL and fatigue scores at week 24, which were maintained through week 52. Statistically significant benefit was also reported in sleep for both doses and % overall work impairment for Sarilumab 150 mg q2w dose.

Table 1. HRQoL, Fatigue, WPAI, and Sleep-VAS at baseline, week 12 (for WPAI only), and week 24:

| HRQoL | Placebo | Sarilumab 150mg + MTX | Sarilumab 200mg + MTX |
|---|---|---|---|
| **SF-36 PCS** Baseline mean (SD) Mean change from BL (SD) p-value | 32.15 **5.27** | 31.92 **8.16** <0.0001 | 31.24 **8.83** <0.0001 |
| **SF-36 MCS** baseline mean (SD) Mean change from BL (SD) p-value | 37.82 3.98 | 39.46 **5.1** 0.02 | 38.92 **7.79** <0.0001 |
| **SF-36 PF** baseline mean (SD) Mean change from BL (SD) p-value | 29.06 4.97 | 29.36 **7.19** 0.0029 | 28.70 **7.77** 0.0008 |

(continued)

| | | | |
|---|---|---|---|
| **SF-36 RP** | | | |
| baseline mean (SD) | 31.93 | 32.37 | 32.03 |
| Mean change from BL (SD) | 4.99 | **7.10** | **7.92** |
| p-value | | 0.0009 | <0.0001 |
| **SF-36 BP** | | | |
| baseline mean (SD) | 33.13 | 33.20 | 32.65 |
| Mean change from BL (SD) | **6.59** | **10.75** | **12.02** |
| p-value | | <0.0001 | <0.0001 |
| **SF-36 GH** | | | |
| baseline mean (SD) | 35.04 | 35.41 | 34.13 |
| Mean change from BL (SD) | 3.83 | **6.11** | 7.73 |
| p-value | | 0.0002 | <0.0001 |
| **SF-36 VT** | | | |
| baseline mean (SD) | 40.67 | 41.30 | 40.19 |
| Mean change from BL (SD) | **5.43** | **7.16** | **9.72** |
| p-value | | 0.0066 | <0.0001 |
| **SF-36 SF** | | | |
| baseline mean (SD) | 34.38 | 35.59 | 34.76 |
| Mean change from BL (SD) | 4.50 | **7.11** | **9.12** |
| p-value | | <0.0001 | <0.0001 |
| **SF-36 RE** | | | |
| baseline mean (SD) | 30.70 | 31.41 | 31.49 |
| Mean change from BL (SD) | 4.50 | **6.21** | **7.70** |
| p-value | | 0.0263 | <0.0001 |
| **SF-36 MH** | | | |
| baseline mean (SD) | 37.07 | 39.15 | 37.59 |
| Mean change from BL (SD) | 4.29 | **5.10** | **7.77** |
| p-value | | 0.0318 | <0.0001 |
| **FACIT-F** | | | |
| Baseline mean (SD) | 27.24 | 22.07 | 26.16 |
| Mean change from BL (SD) | **6.49** | **9.3** | **10.16** |
| p-value | | <0.0001 | <0.0001 |
| **WPAI- % overall work impairment due to RA** | | | |
| Baseline mean (SD) | 51.55 | 49.26 | 54.33 |
| Mean change from BL (SD) | -9.26 | -17.84 | -18 |
| p-value | | 0.0127 | 0.0631 |
| **Sleep VAS** | | | |
| Baseline mean (SD) | 54.05 | 53.77 | 54.23 |
| Mean change from BL (SD) | -16.89 | **-23.17** | **-23.07** |
| p-value | | 0.0008 | 0.0006 |
| PCS=Physical Component Summary, MCS=Mental Component Summary, PF=Physical Function, RP=Role Physical, BP=Bodily Pain, GH=General Health, VT=Vitality, SF=Social Functioning, RE=Role Emotional. MH=Mental Health & VAS=Visual Analog Scale | | | |

## Claims

1. An antibody for use in a method of improving Health-Related Quality of Life of a subject suffering from rheumatoid arthritis,

   wherein the antibody comprises the heavy chain variable region of SEQ ID NO: 1 and the light chain variable region sequence SEQ ID NO: 2, and
   wherein the subject achieves after at least 24 weeks of treatment with the antibody an improvement in Health-Related Quality of Life **characterized by** a change from baseline (BL) in Functional Assessment of Chronic Illness Therapy Fatigue (FACIT-F) of at least 3.

2. The antibody for use of claim 1, wherein the subject achieves a change from BL in FACIT-F of at least 6.5.

3. The antibody for use of claim 1, wherein the subject achieves a change from BL in FACIT-F of at least 9.

4. The antibody for use of claim 1, wherein the subject was previously ineffectively treated for rheumatoid arthritis by administering at least one disease-modifying anti-rheumatic drug (DMARD).

5. The antibody for use of claim 4, wherein the at least one DMARD is selected from the group consisting of methotrexate, leflunomide, sulfasalazine, hydroxychloroquine, abatacept, rituximab, tocilizumab, adalimumab, certolizumab pegol, etanercept, golimumab and infliximab.

6. The antibody for use of claim 4, wherein the at least one DMARD is selected from the group consisting of methotrexate, leflunomide, sulfasalazine and hydroxychloroquine.

7. The antibody for use of claim 4, wherein the at least one DMARD is selected from the group consisting of adalimumab, certolizumab pegol, etanercept, golimumab and infliximab.

8. The antibody for use of any of claims 1 to 7, comprising administering to the subject an effective amount of the antibody and an effective amount of methotrexate, leflunomide, sulfasalazine and/or hydroxychloroquine.

9. The antibody for use of any of claims 1 to 8, comprising administering to the subject an effective amount of the antibody and an effective amount of methotrexate.

10. The antibody for use of claim 9, wherein the methotrexate is administered in an amount of between 6 to 25 mg per week.

11. The antibody for use of claim 9, wherein the antibody and the methotrexate are administered together.

12. The antibody for use of any of claims 1 to 11, wherein the antibody is administered in an amount of between about 100 mg and about 200 mg every two weeks.

13. The antibody for use of any of claims 1 to 12, wherein the antibody is administered in an amount of about 150 mg or about 200 mg every two weeks.

14. The antibody for use of any of claims 1 to 13, wherein the antibody is administered subcutaneously.

15. The antibody for use of any of claims 1 to 14, wherein the antibody is sarilumab.

Europäisches
Patentamt

European
Patent Office

Office européen
des brevets

# EUROPEAN SEARCH REPORT

**Application Number**

EP 21 17 9229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2012/171123 A1 (MEDICH JOHN R [US] ET AL) 5 July 2012 (2012-07-05) * Examples 6, 30, 40; [0426, 0296] * ----- | 1-15 | INV. A61K39/395 A61K31/519 C07K16/28 A61K31/42 |
| X | US 2013/149310 A1 (JASSON MARTINE [FR] ET AL) 13 June 2013 (2013-06-13) * claims 1-20; [0254; 0257] * ----- | 1-15 | A61K31/655 A61K45/06 A61K39/00 A61P29/00 |
| Y | WO 2013/053751 A1 (SANOFI SA [FR]; REGENERON PHARMA [US]) 18 April 2013 (2013-04-18) * claims 1-17 * ----- | 1-15 | A61P19/02 A61P43/00 A61P37/06 A61K31/4706 |
| Y | Barbara Gandek ET AL: "Psychometric evaluation of the SF-36 health survey in Medicare managed care", Health care financing review, 1 January 2004 (2004-01-01), page 5, XP055155378, United States Retrieved from the Internet: URL:http://www.ncbi.nlm.nih.gov/pubmed/15493441 [retrieved on 2014-11-27] * abstract, figure 1; tables 1-6; page 19-23 * ----- | 1-15 | **TECHNICAL FIELDS SEARCHED (IPC)** A61K C07K |
| A | WO 2014/071105 A1 (INFINITY PHARMACEUTICALS INC [US]) 8 May 2014 (2014-05-08) * [00330], [00639]; claims 1-66 * ----- | 1-15 | |

-/--

The present search report has been drawn up for all claims

| Place of search | Date of completion of the search | Examiner |
|---|---|---|
| Munich | 10 December 2021 | Klee, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SALAFFI FAUSTO ET AL: "The health-related quality of life in rheumatoid arthritis, ankylosing spondylitis, and psoriatic arthritis: a comparison with a selected sample of healthy people", HEALTH AND QUALITY OF LIFE OUTCOMES, BIOMED CENTRAL, LONDON, GB, vol. 7, no. 1, 18 March 2009 (2009-03-18), page 25, XP021050341, ISSN: 1477-7525, DOI: 10.1186/1477-7525-7-25 * page 2 - page 10 * | 1-15 | |

TECHNICAL FIELDS
SEARCHED      (IPC)

The present search report has been drawn up for all claims

| Place of search | Date of completion of the search | Examiner |
|---|---|---|
| Munich | 10 December 2021 | Klee, Barbara |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

               

& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 21 17 9229

This annex lists the patent family members relating to the patent documents cited in the above-mentioned European search report.
The members are as contained in the European Patent Office EDP file on
The European Patent Office is in no way liable for these particulars which are merely given for the purpose of information.

10-12-2021

| Patent document cited in search report | | Publication date | Patent family member(s) | | Publication date |
|---|---|---|---|---|---|
| US 2012171123 | A1 | 05-07-2012 | US | 2008131374 A1 | 05-06-2008 |
| | | | US | 2012171123 A1 | 05-07-2012 |
| US 2013149310 | A1 | 13-06-2013 | US | 2013149310 A1 | 13-06-2013 |
| | | | US | 2018296670 A1 | 18-10-2018 |
| WO 2013053751 | A1 | 18-04-2013 | AR | 088289 A1 | 21-05-2014 |
| | | | AU | 2012323062 A1 | 01-05-2014 |
| | | | BR | 112014008764 A2 | 13-06-2017 |
| | | | CA | 2851751 A1 | 18-04-2013 |
| | | | CL | 2014000926 A1 | 21-11-2014 |
| | | | CN | 104105505 A | 15-10-2014 |
| | | | CN | 108310376 A | 24-07-2018 |
| | | | CO | 6950482 A2 | 20-05-2014 |
| | | | DK | 2766039 T3 | 05-03-2018 |
| | | | EP | 2766039 A1 | 20-08-2014 |
| | | | ES | 2660112 T3 | 20-03-2018 |
| | | | HU | E036561 T2 | 30-07-2018 |
| | | | JP | 6122018 B2 | 26-04-2017 |
| | | | JP | 6336171 B2 | 06-06-2018 |
| | | | JP | 2014530226 A | 17-11-2014 |
| | | | JP | 2017137338 A | 10-08-2017 |
| | | | KR | 20140123474 A | 22-10-2014 |
| | | | KR | 20190014117 A | 11-02-2019 |
| | | | LT | 2766039 T | 10-04-2018 |
| | | | MA | 35620 B1 | 01-11-2014 |
| | | | MX | 353126 B | 20-12-2017 |
| | | | NO | 2766039 T3 | 21-04-2018 |
| | | | PL | 2766039 T3 | 29-06-2018 |
| | | | PT | 2766039 T | 26-02-2018 |
| | | | RU | 2014118741 A | 20-11-2015 |
| | | | SG | 10201607215S A | 28-10-2016 |
| | | | SG | 10201807042Y A | 27-09-2018 |
| | | | SG | 11201401373R A | 29-05-2014 |
| | | | SI | 2766039 T1 | 31-05-2018 |
| | | | TR | 201802387 T4 | 21-03-2018 |
| | | | TW | 201321017 A | 01-06-2013 |
| | | | US | 2014302053 A1 | 09-10-2014 |
| | | | US | 2021230719 A1 | 29-07-2021 |
| | | | UY | 34387 A | 31-05-2013 |
| | | | WO | 2013053751 A1 | 18-04-2013 |
| WO 2014071105 | A1 | 08-05-2014 | EP | 2914295 A1 | 09-09-2015 |
| | | | US | 2014120060 A1 | 01-05-2014 |
| | | | US | 2016022692 A1 | 28-01-2016 |
| | | | WO | 2014071105 A1 | 08-05-2014 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 21 17 9229

This annex lists the patent family members relating to the patent documents cited in  the above-mentioned European search report.
The members are as contained in the European Patent Office EDP file on
The European Patent Office is in no way liable for these particulars which are merely given for the purpose of information.

10-12-2021

| Patent document cited in search report | Publication date | Patent family member(s) | Publication date |
|---|---|---|---|

-----------------------------------------------------------------------

EPO FORM P0459

For more details about this annex : see Official Journal of the European Patent Office, No. 12/82

page 2 of 2

## REFERENCES CITED IN THE DESCRIPTION

**Patent documents cited in the description**

- US 6629949 B
- US 6659982 B

**Non-patent literature cited in the description**

- *British Journal of Rheumatology,* 1997, vol. 36, 884-888
- **WARE, JE. ; KOSINSKI, M. ; KELLER, SD.** SF-36 Physical and Mental Health Summary Scales: A User's Manual. The Health Institute, 1994
- **WARE, JE et al.** *MCS and PCS derived from the eight scales,* 1994
- Remington's Pharmaceutical Sciences. Mack Publishing Company
- Compendium of excipients for parenteral formulations. **POWELL et al.** J Pharm Sci Technol. PDA, 1998, vol. 52, 238-311
- **WU et al.** *J. Biol. Chem.,* 1987, vol. 262, 4429-4432
- **LANGER.** *Science,* 1990, vol. 249, 1527-1533
- **MEEHAN et al.** *J. Controlled Release,* 1996, vol. 46, 107-116

SEQUENCE LISTING

<110>   SANOFI BIOTECHNOLOGY

<120>   COMPOSITIONS FOR  IMPROVING THE HEALTH RELATED QUALITY OF LIFE OF
         RHEUMATOID ARTHRITIS PATIENTS

<130>   FR2014-008

<160>   2

<170>   PatentIn version 3.5

<210>   1
<211>   116
<212>   PRT
<213>   Homo sapiens

<220>
<221>   MISC_FEATURE
<222>   (1)..(116)
<223>   heavy chain variable region of sarilumab

<400>   1

```
Glu Val Gln Leu Val Glu Ser Gly Gly Gly Leu Val Gln Pro Gly Arg
1               5                   10                  15


Ser Leu Arg Leu Ser Cys Ala Ala Ser Arg Phe Thr Phe Asp Asp Tyr
            20                  25                  30


Ala Met His Trp Val Arg Gln Ala Pro Gly Lys Gly Leu Glu Trp Val
            35                  40                  45


Ser Gly Ile Ser Trp Asn Ser Gly Arg Ile Gly Tyr Ala Asp Ser Val
        50                  55                  60


Lys Gly Arg Phe Thr Ile Ser Arg Asp Asn Ala Glu Asn Ser Leu Phe
65                  70                  75                  80


Leu Gln Met Asn Gly Leu Arg Ala Glu Asp Thr Ala Leu Tyr Tyr Cys
                85                  90                  95


Ala Lys Gly Arg Asp Ser Phe Asp Ile Trp Gly Gln Gly Thr Met Val
            100                 105                 110


Thr Val Ser Ser
            115
```

<210>   2
<211>   107
<212>   PRT
<213>   Homo sapiens

```
<220>
<221>  MISC_FEATURE
<222>  (1)..(107)
<223>  light chain variable region of sarilumab

<400>  2

Asp Ile Gln Met Thr Gln Ser Pro Ser Ser Val Ser Ala Ser Val Gly
1               5               10              15


Asp Arg Val Thr Ile Thr Cys Arg Ala Ser Gln Gly Ile Ser Ser Trp
        20              25              30


Leu Ala Trp Tyr Gln Gln Lys Pro Gly Lys Ala Pro Lys Leu Leu Ile
        35              40              45


Tyr Gly Ala Ser Ser Leu Glu Ser Gly Val Pro Ser Arg Phe Ser Gly
    50              55              60


Ser Gly Ser Gly Thr Asp Phe Thr Leu Thr Ile Ser Ser Leu Gln Pro
65              70              75              80


Glu Asp Phe Ala Ser Tyr Tyr Cys Gln Gln Ala Asn Ser Phe Pro Tyr
            85              90              95


Thr Phe Gly Gln Gly Thr Lys Leu Glu Ile Lys
            100             105
```